# EUROPEAN PATENT SPECIFICATION

(11) **EP 1 177 790 B1**
(45) Date of publication and mention of the grant of the patent: **11.05.2005**
(21) Application number: 00976087.7
(22) Date of filing: 22.11.2000
(51) Int. Cl.: A61K 31/352, A61K 35/78, A61P 35/00, A61P 25/00

(54) **THERAPY WITH CANNABINOIDS IN THE TREATMENT OF CEREBRAL TUMOR**
THERAPEUTISCHE ANWENDUNG VON CANNABINOIDEN ZUR HIRNTUMORBEHANDLUNG
THERAPIE FAISANT APPEL A DES CANNABINOIDES POUR LE TRAITEMENT DE TUMEURS DU CERVEAU

(30) Priority: 11.02.2000 ES 200000323
(43) Date of publication of application: 06.02.2002
(73) Proprietor: UNIVERSIDAD COMPLUTENSE DE MADRID, E-28040 Madrid (ES)
(72) Inventor: GUZMAN PASTOR, Manuel, Facultad Cc. Biologicas, E-28040 Madrid (ES); SANCHEZ GARCIA, Cristina, Facultad Cc. Biologicas, E-28040 Madrid (ES); GALVE ROPERH, Ismael, Facultad Cc. Biologicas, E-28040 Madrid (ES)
(74) Representative: Carpintero Lopez, Francisco
(86) International application number: PCT/ES2000/000450
(87) International publication number: WO 2001/058445

(56) References cited:
- HARRIS L.S. ET AL.: 'Retardation of tumor growth by delta-9 tetrahydrocannabinol' THE PHARMACOLOGIST vol. 16, no. 2, 1974, page 259, XP000957658
- BRAUDE M.C. & STEPHEN SZARA: 'Pharmacology of marihuana', 1976, RAVEN PRESS, NEW YORK, NY, USA XP000957683 Proceedings of the meeting Savanah, GA, USA, Dic. 3-6, 1974. Illus., vol 1 y 2-A monograph of the national institute of drug abuse, ISBN 0-89004-067-2, HARRIS, L.S. et al.: "Anti-tumor ..." * page 749 - page 762 *
- BAEK SEUNG-HWA, ET AL.: 'Synthesis and antitumor activity of cannabigerol' ARCHIVES OF PHARMACOL. RES. vol. 19, no. 3, 1996, SEOUL, pages 228 - 230, XP000979791
- BAEK SEUNG-HWA, ET AL.: 'Boron trifluoride etherate on silica-A modified Lewis acid reagent (VII). Antitumor activity of cannabigerol against human oral epitheloid carcinoma cells' ARCHIVES OF PHARMACOL. RES. vol. 21, no. 3, June 1998, SEOUL, pages 353 - 356, XP000957635
- RUIZ L. ET AL.: 'Delta-9 tetrahydrocannabinol induces apoptosis in human prostate PC-3 cells via a receptor-independent mechanism' FEBS LETT. vol. 458, no. 3, 1999, pages 400 - 404, XP000981226

## Description

### TECHNICAL FIELD OF THE INVENTION

The technical field of the present invention is the treatment of brain tumors (glioblastomas).

### OBJECT OF THE INVENTION

The present invention relates to a therapeutic use of cannabinoid compounds for treatment of brain tumors. Currently employed therapies for these tumors (surgery, radiotherapy, chemotherapy, immunotherapy, gene therapy) are generally ineffective or at best palliative. The invention implies a technically simple approach lacking appreciable side effects and highly effective in the treatment of brain tumors, including the most malign (glioblastomas).

### BACKGROUND OF THE INVENTION

Among the various brain tumors which affect humans, glioblastomas are the most common (1 per 50,000 persons-year), malign (mortality near 100%) and fastest evolving (life expectation of weeks/months after diagnostic). Nowadays treatment of glioblastomas is generally ineffective or merely palliative, and implies such therapies as surgery, radiotherapy, chemotherapy and immunotherapy (Louis, D.N. & Gusella, J.F., *Trends Genet.* 11, 412-415, 1995; Avgeropoulos, N.G. & Batchelor, T.T., *Oncologist 4,* 209-224, 1999). Additionally, gene therapy is beginning to be used as an experimental treatment for glioblastomas, although so far it has produced few positive results (Martuza, R.L., *Nature Med*. *3,* 1323, 1997). The unlikelihood of success of these therapeutic approaches can be further complicated by factors such as the rapid growth, great heterogeneity, high level of infiltration and an extreme resistance to chemotherapy shown by glioblastomas (Maintz *et al*., *J*. *Neurpathol*. *Exp*. *Neurol.* 56, 1098-1104, 1997; Mason, W, Louis, D.N. & Cairncross J.G. *J. Clin*. *Oncol*. 15, 3423-3426, 1997; Martruza, *op cit*.; Avregopoulos & Batchelor, *op cit.*)*.* It would therefore be highly desirable to develop novel therapeutic alternatives for treatment of brain tumors.

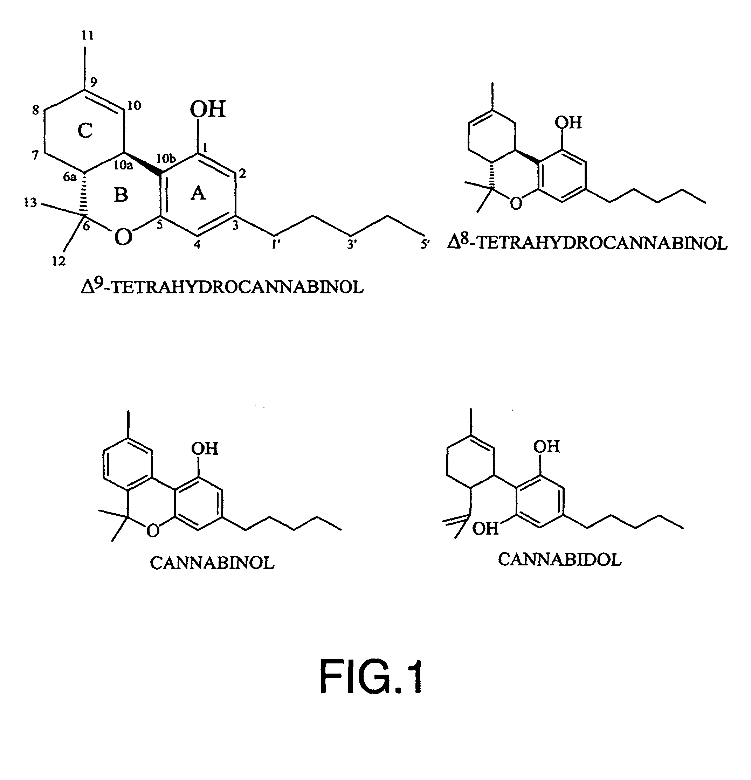

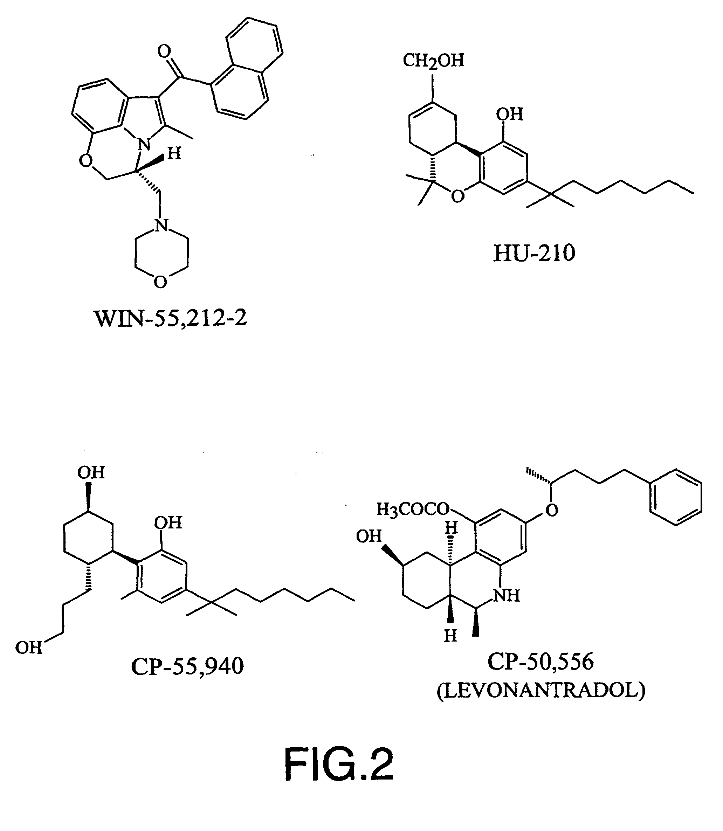

Cannabinoids are compounds named after the plant which synthesizes them, *Cannabis sativa L.* These compounds, among which Δ⁹-tetrahydrocannabinol (THC) stands out for its high potency and abundance, are responsible for the central and peripheral effects of consuming marihuana (Pertwee, R.G:, *Pharmacol. Ther*. 74, 129-180, 1997; Felder, C.C. 6 Glass, M., *Annu*. *Rev*. *Pharmacol*. *Toxicol.* 38, 179-200, 1998). Cannabinoids from *C*. *sativa* (Fig. 1) act by virtue of their similarity to certain molecules produced by animals (including humans) which probably perform important functions in the nervous system. These molecules are therefore known as endogenous cannabinoids or endocannabinoids, among which anandamide (=araquidonoylethanolamide) is the most representative (Di Marzo, V., Melck, D., Bisogno, T. & De Petrocellis, L., *Trends Neurosci.* 21, 521-528, 1998; Martin B.R., Mechoulam, R. & Razdan, R.K., *Life Sci.* 65, 573-595, 1999). Furthermore, compounds have been obtained in the laboratory which mimic the action of natural cannabinoids but with a much higher potency. These are known as synthetic cannabinoids, one of which is WIN-55, 212-2 (Fig. 2) (Pertwee *op cit.;* Barth, F. *Expert Opin*. *Ther*. *Patents,* 8, 301-313, 1998). Both natural and synthetic cannabinoids act by bonding to specific membrane receptors (cannabinoid or CB type receptors), of which two different sub-types are now known: CB₁ and CB₂. (Pertwee, *op cit*., Howlett A. *et al.* in *The IUPHAR Compendium of Receptor Characterization and Classification*, eds. Godfraind, T., Humphrey, P., Ruffolo, R. & Vanhoutte, P., IUPHAR Media, 97-104, 1998). Not all tissues in the organism have these receptors; they are mainly found in the nervous system, and thus the effects of cannabinoids are mainly on the brain (Pertwee, *op cit.* Childers, S.R. & Breivogel, C.S. *Drug Alcohol Depen.* 51, 173-187, 1999).

There are currently a great number of studies which deal with the possible therapeutic applications of cannabinoids. Indeed, in the United Kingdom and in several states of the United States doctors may prescribe THC or certain synthetic as appetite stimulants and vomit inhibitors in patients with AIDS or cancer treated chronically with chemotherapy (Grinspoon, L. & Bakalar, J.B., *JAMA* 273, 1875-1876, 1995; Voth, E. & Schwartz, R. *Ann. Intern. Med*. 126, 791-798, 1997). Among possible therapeutic uses of cannabinoids the following may be mentioned: (a) as analgesic agents they have been shown to be very effective in alleviating sharp and chronic pain; (b) as agents which reduce motor activity they are being tested nowadays for treatment of disorders associated to Parkinson's disease, Huntington's chorea and multiple sclerosis; (c) as anticonvulsive agents their use in treatment of epilepsy is being studied; (d) as agents which reduce intraocular pressure they could be used in treatment of glaucoma (Voth & Schwartz, *op cit*., Manzanares, J. *et al*., *Trends Pharmacol*. *Sci.,* 20, 287-294, 1999; Pop, E., *Curr*. *Opin*. *CPNS Invest. Drugs* 1, 587-596, 1999; Sanudo-Pena, M.C., Tsou, K. & Walker, J.M., *Life Sci.* 65, 703-713, 1999). Some of these therapeutic uses of cannabinoid compounds have already been patented (see for example US4189491, US6939429, WO9711668, WO9832441 and WO9957105).

One of the most intriguing and unexplored effects of cannabinoids is their ability to inhibit the growth of cells transformed *in vitro.* Thus, it has been shown that several cannabinoids inhibit the proliferation of breast tumor cells MCF-7 (De Petrocellis, L. *et al*., *Proc*. *Natl*. *Academ*. *Sci*. *USA* 95, 8375-8380, 1998), glioblastoma cells C6 (Sánchez, C., Galve-Roperh, I. , Canova, C., Brachet, P. & Guzman, M., *FEBS Lett*. 436, 6-10, 1998) and prostate tumor cells PC-3 (Ruiz, L., Miguel, A.. & Diaz-Laviada I., *FEBS Lett.* 458, 400-404, 1999). However, these findings in culture cell systems have never been observed before *in vivo*, so that their biomedical significance is unknown.

### DESCRIPTION OF THE INVENTION

The present invention makes a novel use of cannabinoids in the treatment of brain tumors, and is based on our original observations of cannabinoid-induced marked regressions (implying a longer life) and even eradication (implying curation) of glioblastomas in laboratory animals. This invention involves a technically simple therapy lacking any significant side effects, and more significantly very effective in the treatment of brain tumors, which as mentioned before cannot be satisfactorily treated nowadays by any other techniques or compounds. The experiments which have led to the present invention are described below.

### Antitumoral action of cannabinoids in rats

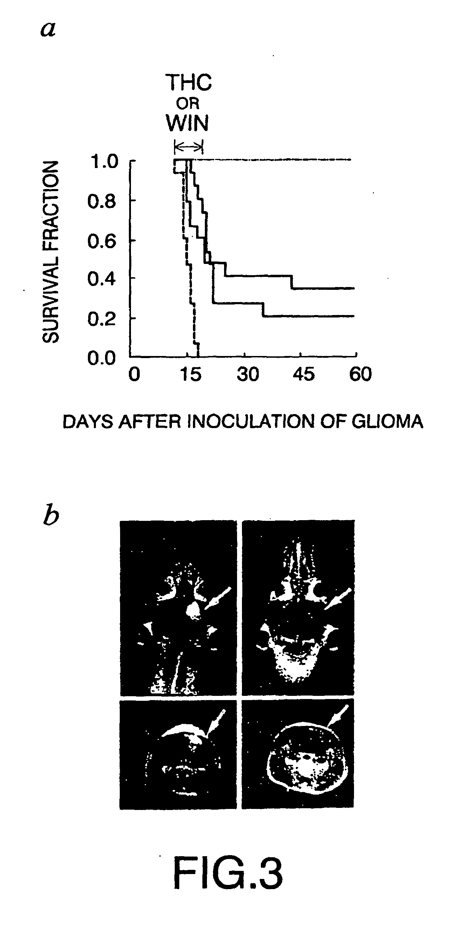

The injection of C6 glioblastoma cells in a rat brain is widely used as an experimental model for a malign brain tumor (Barth, R.F., *J*. *Neurooncol.* 36, 91-102, 1998). C6 glioblastoma cells were directly inoculated in the brain of *Wistar* rats and the tumors viewed by magnetic resonance. All animals left untreated died uniformly 12-18 days after inoculation with the cells (Fig. 3a). To evaluate the antitumoral potential of the cannabinoids, 12 days after inoculating the cells a group of animals was administered THC or WIN-55, 212-2 for 7 days through a cannula located at the site of inoculation. Animals treated with cannabinoids had a significantly longer lifetime than the control animals (Fig. 3a). Thus, administration of cannabinoids managed to increase the survival time to 19-35 days in 9/15 of animals (treatment with THC) or to 19-43 days in 4/15 of animals (treatment with WIN-55, 212-2). Moreover, cannabinoids managed to completely eradicate the tumor in 3/15 of animals(treatment with THC) or 5/15 of animals (treatment with WIN-55, 212-2). Figure 3b shows a magnetic resonance image of one of the animals cured with THC; after administration of the cannabinoid the tumoral mass had disappeared completely, and in its place could be seen a residual hypo intense area interpreted as a fibrous scar in the place of inoculation. No recurrence was observed in any of the 8 animals cured with cannabinoids.

### Antitumoral action of cannabinoids in immunodeficient mice

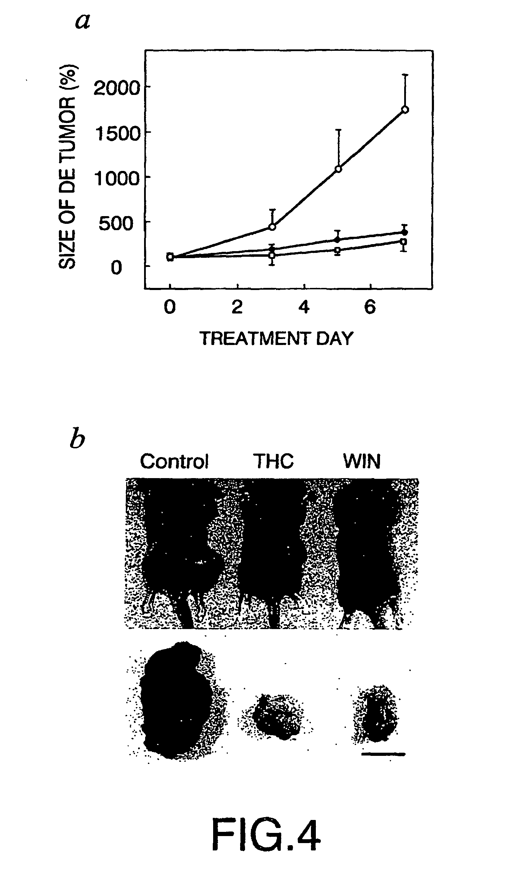

In order to discern whether the antiproliferative action of cannabinoids was due to a direct effect on the tumoral cells or to an indirect effect mediated by an immune response, C6 glioblastoma cells were inoculated subcutaneously in mice with a deficiency of recombinase RAG-2 (RAG-2^{-/-} ), which lack mature T and B lymphocytes (Shinkai *et al*. *Cell* 68, 855-867, 1992). As shown in fig. 4a, the size of the tumors was extraordinarily smaller in animals treated with THC or WIN-55, 212-2 than in the control animals. Figure 4b shows examples of tumor bearing mice and tumors dissected after treatment with or without cannabinoids for 7 days.

### Safety of in vivo treatment with cannabinoids

After this were studied the possible side effects of treatment with cannabinoids. Rats without tumors which were treated with cannabinoids had a fully unaffected survival (Fig. 3a). As with the 8 animals mentioned above whose tumors were eradicated with cannabinoids, a detailed analysis by magnetic resonance of all tumor-less animals revealed that treatment with cannabinoids did not result in any signs of damage by necrosis, edema, infection, inflammation or trauma. To rule out the possibility of toxic effects of cannabinoids on nerve cells undergoing division, TUNEL tinctures were performed in the subventricular area of the brain in rats, which continues to proliferate in the adult animal. Administration of cannabinoids did not only not produce any significant apoptotic effects in the brain *in vivo*, but in addition the slight marking observed in the *caudado putamen* of control animals was not apparent in animals treated with cannabinoids.

In both tumorless animals and tumor bearing animals, cannabinoids did not induce any significant alteration of behavioral parameters such as motor coordination and physical activity. Intake of food and water and weight gain were also unaffected by cannabinoids. Likewise, in blood analyses biochemical parameters (glucose, urea, uric acid, creatinine, cholesterol, bilirubine) and tissue damage markers (alanine and aminotransferase aspartate, γ-glutamyltransferase, creatin, quinase, dehydrogenase lactate) were not affected neither throughout the 7-day period of administration nor up to 2 months after ending the treatment with cannabinoids. Data from other authors support the idea that cannabinoids are not only not toxic compounds for nerve cells, but instead protect them from toxic stimuli such as glutamergic agonists (Skaper *et al*. *Proc*. *Natl*. *Academ. Sci. USA* 93, 3984-3989, 1996; Shen, M. & Thayer, S.A., *Mol. Pharmacol*., 54, 459-462, 1998), oxidative agents (Hampson A.J., Grimaldi M., Axelrod J. & Wink, D. *P Proc*. *Natl*. *Academ*. *Sci*. *USA 95,* 8268-8273, 1998) and ischemia (Nagayama T. *et al*., *J*. *Neurosci*. 19, 2987-2995, 1999).

### Pharmacological characterization of the antitumoral action of cannabinoids

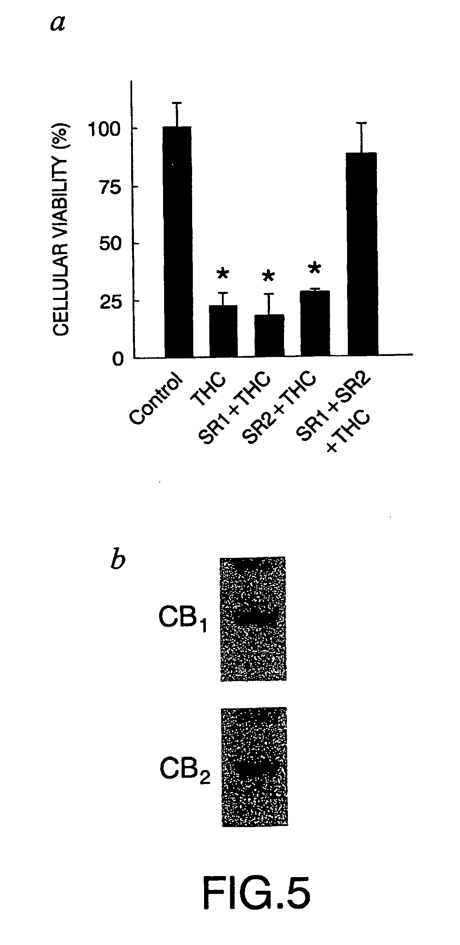

Experiments were conducted with the object of obtaining a pharmacological characterization of cannabinoid-induced death of C6 glioblastoma culture cells. High potency synthetic agonists such as WIN-55,212-2, CP-55,940 and HU-210 induced the death of these cells in lower doses than THC, as could be expected from their greater affinity for cannabinoid receptors (Pertwee, *op cit.*). Thus, after 5 days of exposure to cannabinoids the viability of a C6 glioblastoma was reduced by 50% in concentrations of 20 nM WIN-55,212-2, 45 nM CP-55,940, 10 nM HU-210 and 480 nM THC (n=4). Neither SR141716 (a selective antagonist of CB₁) nor SR144528 (a selective antagonist of CB₂) (Shire, D. *et al*. Life Sci. 65, 627-635, 1999) were separately capable of preventing the cell death induced by the THC. However, when the two antagonists were jointly added to the incubations an effective prevention was observed of the cell death induced by THC (Fig. 5a). In agreement with this a Western blot test showed that C6 glioblastoma cells expressed both receptor CB₁ and CB₂ (Fig. 5b).

### Application of the invention to other cases

The experiments which led to the present invention were conducted with rats and mice as the tumor bearing animals. However, in view of the experimental design used and the similarity of brain tumors in different mammals (R.F. Barth, *op cit.*), the invention can be applied to the treatment of brain tumors in other mammals, including man.

The experiments which led to the present invention were performed with glioblastomas as a model of a brain tumor.

The experiments which led to the present invention were performed with two paradigmatic cannabinoids, a natural one (THC) and a synthetic one (WIN-55,212-2). In a preferred embodiment of the invention the cannabinoid is used with the most potent antiproliferative effect for a given tumor. However, as the antiproliferative effect of these compounds is mediated by cannabinoid receptors (CB type receptors, Howlett *et al*. *op cit.*), the invention is applicable to any other agonist of these receptors, whether cannabinoids from *C*. *sativa* (such as Δ⁹-tetrahydrocannabinol, cannabinol, cannabidiol) (Fig. 1) or synthetic cannabinoids (such as HU-210, CP-55,940, CP-50,556) (Fig. 2) (Pertwee, *op cit.;* F. Barth, *op cit.*). Also included in this section are drugs which contain any cannabinoid in their composition.

The experiments which led to the present invention were performed with the intratumoral administration of the cannabinoid. In a preferred embodiment of the invention this would be the administration form of choice, as it allows a high accessibility of the cannabinoid to the tumor. However, as the action of the cannabinoid is direct on the tumor and does not seem to affect peripheral system the form of administration may also be systemic, such as intraperitoneal, intravenous or oral.

The experiments which led to the present invention were performed with a continuous administration of a dose of cannabinoid for a set time. In a preferred embodiment of the invention these parameters could be altered according to the specific requirements of the treatment: patient status, size and location of the tumor, number of tumors, etc. Thus, for example, the mode of application could be continuous (preferred mode) or sequential in one or several doses per day. This would obviously affect the dose of compound administered and the total time of treatment.

### BRIEF DESCRIPTION OF THE FIGURES

Figure 1
   Chemical formula of the main cannabinoids of *C. sativa.*
Figure 2
   Chemical formula of the main synthetic cannabinoids.
Figure 3
   Antitumoral action of cannabinoids in rats.
   (a) Survival curves for rats with brain tumors. Glioblastomas were induced in 45 rats (day 0); 15 animals were not treated with cannabinoids (―) while another 15 were treated with THC ( ―) and another 15 with WIN55,212-2 (···) between days 12 and 19. The animals treated with cannabinoids lived significantly longer than the control animals (P<0.01 by the log-rank test). THC and WIN-55,212-2 were also administered to 5 rats each without an induced brain tumor (·―·―).
   (b) Magnetic resonance image in axial projection (top) and coronal projection (bottom) of the brain of a rat before (left) and after (right) treatment with THC. A 100 mm³ glioblastoma (arrow) was eradicated by 500 µg of THC. The image was taken 7 days after ending the treatment with THC.
Figure 4.
   Antitumoral action of cannabinoids in immunodeficient mice.
   (a) Glioblastomas were induced in 18 mice. When the tumors reached the desired size (day 0) 6 animals were treated with vehicle ( ) while another 6 were treated with THC ( ) and another 6 with WIN-55,212-2 (=) for 7 days. The size of the tumors in animals treated with cannabinoids was significantly smaller than in control animals at all times (P<0.01 by the Student t test).
   (b) Examples of glioblastomas in mice (top) and dissections (bottom, bar: 1 cm.) after treatment for 7 days with vehicle, THC or WIN-55,212-2 (WIN).
Figure 5
   Implication of cannabinoid receptors in cell death
   (a) C6 glioblastoma cells were cultivated for 5 days in the presence or absence of 1 µM THC, 1 µM SR141716 (SR1) and/or 1 µM SR144528 (SR2) (n=6). *Significantly different from incubations without additions (P<0.01 by the Student t test).
   (b) Presence of cannabinoid receptors CB₁ and CB₂ in C6 glioblastoma cells. Detection of the receptors was made by Western blot with specific antibodies for each of the two receptors.

### EMBODIMENT OF THE INVENTION

The present invention is further illustrated by the examples described below.

### Example 1.

### Curation of glioblastomas in rats

Male Wistar rats (250-300 g in body mass) were anaesthetized with 3% isofluorane in an oxygen mixture (0.8 l/min) and protoxide (0.4 l/min). 5x10⁸ C6 glioblastoma cells were prepared in 100 µl of saline solution buffered with phosphate (PBS) and supplemented with 0.1% glucose, and stereotaxically injected in the frontal parietal lobule of the right hemisphere (4 mm to the right of bregma, 4.5 mm depth from the cranium) (Izquierdo, M *et al*., *Gene Ther.* 2, 66-69, 1995). The rats received dexamethasone (2 mg/l) and tetracycline (75 mg/kg of body weight) in water for 3 days before and 7 days after inoculation of the cells. A thorough monitoring of the tumors was performed by magnetic resonance with the methods described by other authors (Izquierdo, M *et al*., *op cit;* Cortés, M.L., de Felipe, P., Martin, V. Hughes, M.A. & Izquierdo, M. *Gene Ther.* 5, 1499-1507, 1998).

The administration of cannabinoids to rats began 12 days after inoculation of the cells. At this time the average size of the tumors was 70 mm³ (interval 25-100 mm³) as estimated by magnetic resonance (Izquierdo, M *et al*., *op cit;* Cortés, de Felipe, Martin, Hughes & Izquierdo, M, *op cit.*)*.* Cannabinoids were administered by a cannula placed at the place of inoculation of the tumor and attached to the cranium by dental cement; a small stainless steel screw anchored the cannula and the dental cement. The cannula was subcutaneously connected by a catheter to an osmotic mini-pump (Alzet 2001) which operated at a flow of 1 µl/h for 7 days. The osmotic pump was filled with 500-2500 µg of THC or 50-250 µg of WIN-55,212-2 in 200 µl of PBS supplemented with 5 mg/l of bovine serum albumin (BSA), delipidized and dialyzed. The dose of cannabinoid employed depended on the characteristics of the tumor to be treated. Greater doses were used for large, dense and invasive tumors.

As seen in figure 3a, all animals left untreated died uniformly within 12-18 days after inoculation of the cells. The animals treated with cannabinoids had a significantly longer life than control animals. Furthermore, cannabinoids fully eradicated the tumor in a significant percentage of animals. Figure 3b shows a magnetic resonance image of one of the animals cured with THC; after administering the cannabinoid the tumoral mass disappeared completely and a residual hypointense area is observed which is interpreted as a fibrous scar at the place of inoculation. No recurrence was observed in animals cured with cannabinoids.

### Example 2.

### Curation of glioblastomas in immunodeficient rats.

Tumors were induced in RAG-2^{-/-} rats by subcutaneous inoculation of 5x10⁶ C6 glioblastoma cells in 100 µl of PBS supplemented with 0.1% glucose. About 10 days later, when the average volume of the tumors was 250 mm3 (interval 200-300 mm³) the animals were divided randomly into 3 groups and they were injected during 7 days with vehicle, 500 µg of THC or 50 µg of WIN-55,212-2 per day in 100 µl of PBS supplemented with 5 mg/ml of delipidized and dialyzed BSA. The tumor sizes were measured by a caliper and their volume calculated according to (4π/3) x (width/2)² x (length/2). As shown in Fig. 4a, the size of tumors was much smaller in animals treated with THC or WIN-55,212-2 than in control animals. In Fig.4b are shown examples of tumor bearing mice and tumors dissected after treatment with or without cannabinoids for 7 days.

### Example 3

### Implication of cannabinoid receptors in the death of glioblastoma cells.

C6 glioblastoma cells were cultivated at 37°C and 5%CO₂ in F-12 medium supplemented with calf fetal serum at 10%. 24 h before the start of the experiment the cells were transferred to an F-12 medium free of serum and supplemented with insulin (5 µg/ml), transferrine (10 µg/ml), sodium selenite (5 µg/ml) and delipidized and dialyzed BSA (10 mg/ml). The medium was renovated every 48 h and the cell viability was determined by the MTT method (Sanchez, C., Galve-Roperh, I., Canova, C., Brachet, P. & Guzman, M., *op cit.*)*.* As shown in Fig. 5a, THC was capable of inducing the death of C6 glioblastoma cells. Additionally, when SR141716 (a selective antagonist of CB₁) and SR144528 (a selective antagonist of CB₂) were simultaneously added to the medium the death cell induced by THC was prevented.

In order to confirm that both receptors were present in the C6 cells the cells were washed with PBS, the plates scraped in lysis medium and the particulate fraction obtained by centrifuging at 40,000g for 60 min. (Sanchez, C., Galve-Roperh, I., Canova, C., Brachet, P. & Guzman, M., *op cit*.). The samples were subjected to electrophoresis in polyacrylamide gel with dodecil sodium sulphate and the proteins transferred from the gels to nitrocellulose membranes. The membranes were blocked with delipidized and dialyzed BSA at 1% and incubated with an antibody for residues 1-14 of the CB₁ rat receptor (diluted 1:5000) or with an antibody for residues 350-361 of the CB₂ human receptor (diluted 1:2000). The samples were finally subjected to developing with an electrochemoluminescence kit (Amersham, Bucks, United Kingdom). As shown in Fig. 5b, C6 glioblastoma cells expressed both receptor CB₁ and receptor CB₂.

### Example 4

### Safety of in vivo treatment with cannabinoids.

Cannabinoids were administered (2500 µg of THC or 250 µg of WIN-55,212-2) to tumor-less rats for 7 days as described previously. The rats were then sacrificed and their brains fixed with 4% paraformaldehyde in PBS. Death by apoptosis was determined in 40 µm thick brain slices using a TUNEL tincture kit in accordance with the supplier's instructions (Boehringer, Mannheim, Germany). Marking of DNA strands with triphosphate deoxyuridine marked with fluoresceine was visualized with a confocal microscope (excitation wavelength 488 nm, emission wavelength 525 nm). The laser intensity and the sensitivity of the photodetector were kept constant to allow a comparison of the treatments. At least 5 optical fields were analyzed per animal.

TUNEL tinctures were performed in the subventricular area of the rat brains, which continues to proliferate in the adult animal. Administration of cannabinoids not only did not cause any significant apoptotic effect in the *in vivo* brain, but additionally the slight marking observed in the *caudado putamen* of the control animals was not observed in the animals treated with cannabinoids.

## Claims

1. Use of natural and synthetic cannabinoids in the manufacture of a drug for therapeutic treatment in mammals, including man, of glioblastomas.

2. Use according to claim 1, in which the natural cannabinoids are chosen from the group comprising Δ⁹-tetrahydrocannabinol (THC), Δ⁸-tetrahydrocannabinol, cannabinol and cannabidiol.

3. Use according to any of the above claims in which the natural cannabinoid is Δ⁹-tetrahydrocannabinol (THC).

4. Use according to claim 1, in which the synthetic cannabinoids are chosen from the group comprising WIN-55,212-2, HU-210, CP-55,940 and CP-50,556 (levonantradol).

5. Use according to claims 1 and 4, in which the synthetic cannabinoid is WIN-55,212-2.

## Patentansprüche

1. Verwendung von natürlichen und synthetischen Cannabinoiden bei der Herstellung eines Medikaments zur therapeutischen Behandlung von Glioblastomen bei Säugetieren, einschliesslich Menschen

2. Verwendung gemäss Anspruch 1, wobei die natürlichen Cannabinoide aus der Gruppe gewählt werden, die Δ⁹-Tetrahydrocannabinol (THC), Δ⁸-Tetrahydrocannabinol, Cannabinol und Cannabidinol umfasst.

3. Verwendung gemäss einem der vorangegangenen Ansprüche, wobei das natürliche Cannabinoid Δ⁹-Tetrahydrocannabinol (THC) ist.

4. Verwendung gemäss Anspruch 1, wobei die synthetischen Cannabinoide aus der Gruppe gewählt werden, die WIN-55,212-2, HU-210, CP-55,940 und CP-50,556 (levonantradol) umfasst.

5. Verwendung gemäss Anspruch 1 und 4, wobei das synthetische Cannabinoid WIN-55,212-2 ist.

## Revendications

1. Utilisation de cannabinoïdes naturels et synthétiques dans la fabrication d'une drogue pour le traitement thérapeutique chez les mammifères, y compris l'homme, de glioblastomes.

2. Utilisation selon la revendication 1 dans laquelle les cannabinoïdes naturels sont choisis parmi le groupe comprenant le Δ⁹-tétrahydrocannabinol (THC), le Δ⁸- tétrahydrocannabinol, le cannabinol et le cannabidiol.

3. Utilisation selon l'une quelconque des revendications antérieures dans laquelle le cannabinoïde naturel est le Δ⁹-tétrahydrocannabinol (THC).

4. Utilisation selon la revendication 1, dans laquelle les cannabinoïdes synthétiques sont choisis parmi le groupe comprenant WIN-55.212-2, HU-210, CP-55.940 et CP-50.556 (levonantradol).

5. Utilisation selon les revendications 1 et 4, dans lesquelles le cannabinoïde synthétique est le WIN-55.212-2
